(19) Europäisches Patentamt

European Patent Office

Office européen des brevets

(11) **EP 4 449 436 B1**

(12) **EUROPEAN PATENT SPECIFICATION**

(45) Date of publication and mention
of the grant of the patent:
**02.04.2025 Bulletin 2025/14**

(21) Application number: **23833094.8**

(22) Date of filing: **15.12.2023**

(51) International Patent Classification (IPC):
**G16H 30/40** (2018.01)      **A61B 6/00** (2024.01)
**G16H 40/60** (2018.01)      **G06T 11/00** (2006.01)

(52) Cooperative Patent Classification (CPC):
**G16H 30/40; A61B 6/4405; A61B 6/5258;**
**G16H 40/60;** A61B 6/0492; A61B 6/547

(86) International application number:
**PCT/EP2023/086128**

(87) International publication number:
**WO 2024/132957 (27.06.2024 Gazette 2024/26)**

(54) **METHOD FOR MOBILE X-RAY ACQUISITION OF A PATIENT**

VERFAHREN ZUR MOBILEN RÖNTGENAUFNAHME EINES PATIENTEN

PROCÉDÉ D'ACQUISITION DE RAYONS X MOBILES D'UN PATIENT

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **22.12.2022 RU 2022133852**

(43) Date of publication of application:
**23.10.2024 Bulletin 2024/43**

(73) Proprietor: **Koninklijke Philips N.V.**
**5656 AG Eindhoven (NL)**

(72) Inventors:
• **STADELMANN, Joël, Valentin**
**5656 AG Eindhoven (NL)**
• **SIRAZITDINOV, Ilyas**
**5656AG Eindhoven (NL)**

(74) Representative: **Philips Intellectual Property &**
**Standards**
**High Tech Campus 52**
**5656 AG Eindhoven (NL)**

(56) References cited:
**WO-A1-2021/150474      US-B2- 10 743 822**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The invention relates to the field of X-ray imaging of a patient, and more specifically to a method for mobile X-ray acquisition of a patient, a system for mobile X-ray acquisition, a software module for mobile x-ray acquisition, and a use of a marker used in the method and/or the system.

BACKGROUND OF THE INVENTION

Mobile X-ray systems are often used in emergency departments and intensive care units since a mobile X-ray system may be the only imaging modality for bedside patients. Usually, the quality of the mobile X-ray images is lower than the image quality of a stationary X-ray system. Further, the relative position of the emitter and of the detector are flexible in mobile X-ray systems in order to accommodate life support devices. In X-ray images obtained by a mobile X-ray system, objections visible in the X-ray image may appear different depending on how the X-ray source is positioned relative to the patient. For instance, if the technician is required to place the X-ray source over the head of the patient the object will result in a different silhouette, i.e. Than if, he had placed the X-ray source over the patient's belly. Those distortions complicate physical measurements.

WO 2021/150474 A1 discloses a system forming part of the close prior art of the invention.

SUMMARY OF THE INVENTION

Therefore, there exist a need for optimizing a mobile X-ray acquisition of a patient, more specifically there exist a need for reducing the projection distortion caused by the different positions of the X-ray source over the patient, such that an execution of physical measurements on X-ray images can be enabled.

An object of the invention is to provide an improved method, a system, and a software module and a marker used for mobile X-ray acquisition, wherein in particular the projection distortion may be reduced for enabling execution of physical measurements on X-ray images.

The object of the present invention is solved with the subject matter of the independent claims, wherein further embodiments are incorporated in the dependent claims.

It should be noted that any feature, function and/or element described in the following with reference to the method equally applies to the system and/or the software module, and vice versa. Accordingly, any feature, function, step and/or element described in the following with reference to one aspect of the present disclosure equally applies to any other aspect of the present disclosure.

According to a first aspect of the invention, a method for evaluating a mobile X-ray acquisition of a patient according to claim 1 is described.

In the context of the present invention, the term "mobile X-ray" shall be understood to describe X-ray devices (systems) which are often used in emergency departments and/or intensive care units, which may be used for X-ray imaging bedside patients. The position of the emitter, the X-ray source, and the X-ray detector are flexible, hence not fixed to each other.

In the context of the present invention, the term "known geometry" shall be understood to describe, that the geometry, hence the width, length, height, etc. of the marker is known for the method. It is not important what the exact geometry might be, which means it is not important whether the marker has a height of 10 cm or 20 cm. Instead, it is only important that the geometry is known and provided and not whether the marker has a specific geometry.

In other words, a method is described, which infers the position of the X-ray source relative to the patient by analyzing the projection distortion of a marker of known geometry. The markers silhouette, hence the projection, on the X-ray image may be located using a deep learning based detection method. Further, the knowledge of the position of the X-ray source relative to the patient may be used to recalculate the relative position of important image structure(s). The problem of the projection distortion of mobile X-ray images is solved. Hence, a precise physical measurement on mobile X-ray images may be allowed, for instance a measurement from the endotracheal tube tip inserted in the patient to the carina of the patient, and/or a measurement from central venous catheter tip to superior vena cava region. The method may also help to measure a size of lung nodules. cardiothoracic index etc.

In particular, a method, a system, a software module, and a use of at least one markers is described for evaluation of mobile X-ray images and an improvement thereof, for enabling a determination of the X-ray source above the patient which allows to reduce the influence of the projection distortion and allows to recalculate relative positions of important image structure(s).

It is standard procedure in the acquisition of a chest X-ray to place markers, indicating the patients left or right side. The markers used are the letters "L" and "R" for the respective side of the patient. The marker geometry may be important for the evaluation of the distortion. Therefore, the maker geometry needs to be known, for instance standardized marker may be used, which comprise a standardized dimensions. The used standardized marker is described in further embodiments herein below.

The method (and also the system, the software module and the use of the marker) allows overcoming the problem of projection distortion by adding specific physical markers of a known geometry to the patient for the X-ray imaging, such that these markers may be visible in the X-ray image. Those marker, visible in the X-ray image, may be used for calculation the distortion errors. The resulted errors may be used for estimating corrections for some applied task, e.g. physical measurements on X-ray imaging.

A processing unit may be configured to perform the above mentioned method steps, wherein the processing unit may be part of a computer. The computer may be part of an X-ray imaging system or may be an external computer, which receives all information, in particular, the X-ray images and the instructions for performing the method as described by the different embodiments herein. The processing unit may be configured to perform the method steps one after another, or performing only some of them at the same time or performing all of them simultaneously.

According to an exemplary embodiment of the invention, the step of analyzing the projection of the rod may comprise at least one of the steps of analyzing the projection of the rod along the image X-axis, analyzing the projection of the rod along the image Y-axis, analyzing the projection of the rod along the axis of the rod. The step of analyzing the projection of the rod may comprise one or more of the above mentioned steps, or any combination thereof. In particular, the step of analyzing the projection of the rod may comprises the analysis long the image axes, for obtaining the horizontal displacements on both axes, which can be used for determine the position of the X-ray source above the bed of the patient.

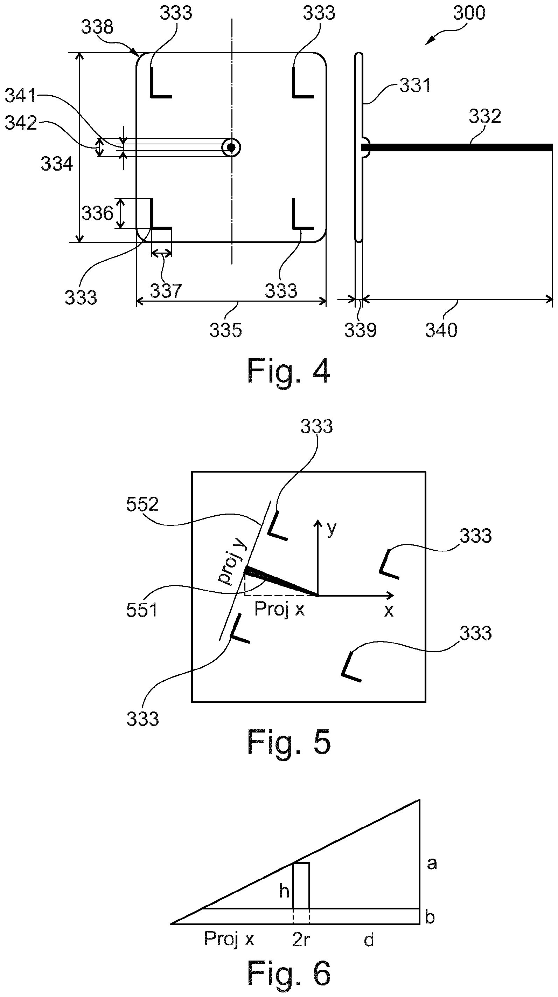

According to an exemplary embodiment of the invention, the step of analyzing the projection of the rod along the image X-axis may comprise measuring the projection of the rod in the X-ray image, and/or determining the rods radius and height from the known geometry of the marker. Further details for analyzing the projection of the rod is described with Fig. 5 to Fig. 7. The known geometry of the marker may be stored in a memory of a processing unit, which is used for performing the method. Hence, when using standardized markers, the dimension of the marker and the rod can be provided from the memory. On the other hand, the geometries and dimensions of the marker and the rod may also be provided by the user for each X-ray acquisition. Accordingly, it is not important from where the dimensions and geometries of the marker and the rod are received, they just need to be known for the method.

According to an exemplary embodiment of the invention, the step of analyzing the projection of the rod along the image Y-axis may comprise measuring the projection of the rod in the X-ray image, and/or determining the rods radius and height from the known geometry of the marker.

According to an exemplary embodiment of the invention, the step of analyzing the projection of the rod along the axis of the rod may comprise determining a lower rod radius at a base of the rod, determining a higher rod radius at the a tip of the rod. It may also be possible that instead of the radius the diameter is used, accordingly the lower diameter and the higher diameter of the rod at its base and tip respectively. The determination of the lower rod radius and the higher rod radius may be carried out at a projection of the rod along the rods direction and not along the image x- or y-axis.

According to an exemplary embodiment of the invention, the method may further comprise the steps of determining a distance from the rod to a line normal to the bed of the patient passing through the X-ray source, and/or determining a distance between the bed of the patient and a detector. The determination of the distances may be carried out using equations as described in the embodiments of Fig. 5 to Fig. 7.

According to an exemplary embodiment of the invention, the projection of the rod, the lower radius of the rod, and the higher radius of the rod may be measured by pixel measurement on the X-ray image and may be converted into physical dimensions using a resolution stored in a DICOM tag.

According to an exemplary embodiment of the invention, the step of detecting the marker may comprise a detection of letters arranged at the marker. In particular, the step may comprise the detection of at least two letters arranged at the marker. Preferably, the step may comprise the detection of all letters, for instance four letters, arranged at the marker. The more letters are detected, the better the determining of the position of the rod can be carried out when based on the letter detection. For instance, in an embodiment the marker comprises four letters arranged in a square pattern on one side of the plate of the marker. Hence, letters do not occur naturally in a body, therefore a detection of the letters in order to locate the markers is proposed. This provides the following advantages. If a letter is occluded (or also two letters, such that only two letters of all four letters can be detected) the marker position can still be inferred using the remaining three or two letters. If a patient may have an orthopedic implant, the implant will not be mistaken for the rod of the marker, because implants do not come with metallic letter around them.

According to an exemplary embodiment of the invention, the marker may comprise four letters arranged at one side of the plate of the marker, wherein the step of detecting the marker may comprise a detection of at least two letters of the marker for determining the position of the rod. If all letters have been detected, the position of the rod may be determined. For example, in the case were all four letters are detected the position of the rod can be determined by the following equation (1):

$$
\begin{aligned}
R_x &= 0.25 \cdot (L_{x1} + L_{x2} + L_{x3} + L_{x4}) \\
R_y &= 0.25 \cdot (L_{y1} + L_{y2} + L_{y3} + L_{y4})
\end{aligned}
\tag{1}
$$

With Rx, Ry being the base position of the rod at the center of the marker plate, Lxi and Lyi being the x and y coordinates of the letters in the X-ray image. In cases were one or two letters are occluded, hence cannot be detected, further rules must be added to assess the position of the letter in the square and deduce the rods base position. For instance, if three letters may be detected, and one letter is occluded, the three letters forming a triangle. The longest distance between the letters have to be determined, which longest distance forms the hypotenuse of the triangle and the base position of the rod can be found in the middle of the hypotenuse of the triangle formed by the letters.

According to an exemplary embodiment of the invention, the deep learning method may use a deep learning network trained with a dataset of letters on X-rays, wherein the data set is trained by letters in different size and rotation. In particular, the data set may be trained by letters of the whole alphabet, hence the letter on the markers are not limited to certain letters. Nevertheless, the preferred letters may be the "L" for indicating a left side of the patient, and a "R" for indicating the right side of a patient in an X-ray image. Further, the data set may be trained with letters as capital letters and lowercase letters. The deep learning method may also be trained on rotated letters, such that every alignment of the letters in the X-ray image can be detected. Hence, the deep learning method for marker detection alleviates the need for the technician to align the marker with the image main axes.

According to an exemplary embodiment of the invention, the method may further comprise the step of calculating a relative position of an image structure based on the determined position of the X-ray source relative to the patient. For instance, simple trigonometric relations can be used in order to determine the actual relative position of a critical image structure. For example, the trachea and the endotracheal tube may be segmented, the projection of the interval between the endotracheal tube tip and carina point can be measured on the X-ray. Then statistical atlases are used to obtain the typical distance between the trachea and the patients bed (the patients back), the actual physical distance between the endotracheal tube tip and the carina point can be recalculated, thus the endotracheal tubes positioning can be confirmed or not.

According to an exemplary embodiment of the invention, the plate of the marker may comprise a square shape, wherein the rod is arranged at the center of the plate, wherein at least two letters are arranged on the plate of the marker, wherein the letters are equal and each letter is arranged in/at one edge of the square shaped plate. The marker may consist of a square plate, or also named base, which can be made of plastic. Further, the marker may comprise for example four inlaid flat letters identifying the marker. The letters may be made of metal. The plate may be made of plastic, in order to minimize its visibility on the received X-ray image. For instance, the dimensions of the plate may be 80 mm width, 80 mm length, and 3 mm in height. The rod of the marker may be arranged at the center of the plate of the marker, wherein the rod may be made of metal and has a cylindrical shape of known height and diameter for instance 80 mm in length and a diameter of 2,5 mm. Further, in the plate a space may be provided, which space can be used for receiving the rod, in which the rod can be clipped by an elastic element and thus ease the transportation to the patients bed. The rod may be attached to the plate by a plastic thread. Further, the plastic parts of the marker may be realized in nylon. The textile structure of nylon may indicate its harmless structure to the patient, when placed in proximity of the patient. Further material properties may that for plastic parts a radiolucent plastic may be used, for metal parts stainless steel and/or radiopaque material such as titanium, aluminum, or tungsten may be used. The exact marker dimensions may not be important, but they may depend on the ease of handling of the marker. Hence, they should be big enough for being carried per hand.

According to an exemplary embodiment of the invention, the step of determining the marker using deep learning method for object detection may comprises in particular a Faster RCNN or YOLO. The letters may be detected on an X-ray image using the deep learning methods, for instance as YOLOv4.

According to a second aspect of the invention, as system for evaluating a mobile X-ray acquisition according to claim 13 is described.

All embodiments as described with the method may also apply to the system for mobile X-ray measurement. The X-ray source may be arranged in any position above the patient, hence it is preferably a mobile X-ray system. The patient may be arranged between the detector and the X-ray source.

According to a third aspect of the invention, a software module according to claim 14 is described. The X-ray image may be received by the computer. On the other hand, the X-ray image may already be included in a memory of the computer, or the X-ray image may be provided to the software module from cloud or directly from the mobile X-ray system.

The software module may be part of a computer and/or computer program, but it can also be an entire program by itself. For example, the software module may be used to update an already existing computer program to get to the present invention.

The software module may be stored on a computer readable medium. The computer readable medium may be seen as a storage medium, such as for example, a USB stick, a CD, a DVD, a data storage device, a hard disk, or any other medium on which a software module as described above can be stored.

According to a fourth aspect of the invention, a use of a marker for evaluating a mobile X-ray acquisition according to claim 15 is described.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

It has to be noted that embodiments of the invention have been described with reference to different subject matters. In particular, some embodiments have been described with reference to apparatus/system type claims whereas other embodiments have been described with reference to method type claims.

BRIEF DESCRIPTION OF THE DRAWINGS

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

Fig 1 illustrates a flow diagram of the method according to an exemplary embodiment of the invention.

Fig. 2 illustrates different projections of an object in dependence of the position of an X-ray source.

Fig. 3 illustrates different projections of different objects using one X-ray source.

Fig. 4 illustrates a marker according to an exemplary embodiment of the invention.

Fig. 5 illustrates an analysis of a projection of the rod according to an embodiment of the invention.

Fig. 6 illustrates an analysis of the projection of the rod along an image axis according to an embodiment of the invention.

Fig. 7 illustrates an analysis of the projection of the rod along the rod axis according to an embodiment of the invention.

DETAILED DESCRIPTION OF EMBODIMENTS

The illustrations in the drawings are schematic. It is noted that in different figures similar or identical elements are provided with the same reference signs.

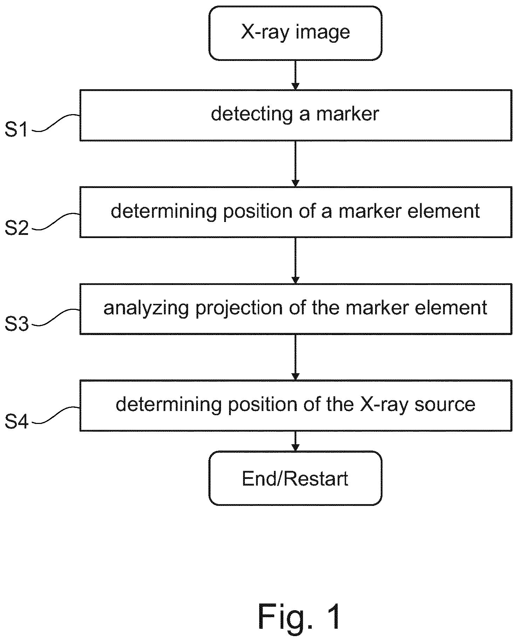

Fig. 1 illustrates a flow diagram comprising method steps according to an embodiment of the invention. The method comprises the step S1 detecting a marker in an X-ray image using a deep learning method, wherein the marker comprises a plate of known geometry and a rod. Further, the method comprises the step S2 determining a position of the rod of the marker, step S3 analyzing a projection of the rod in the X-ray image, and step S4 determining an position of the X-ray source above the patients bed based on the analyzed projection of the rod. The method starts with the detecting of a marker S1, for being able to detect the marker, an X-ray image of the patient is received before the detection of the marker can start. This may be included in step S1. The step S3 of analyzing the projection of the rod may further comprise at least one of the steps of analyzing the projection of the rod along the image X-axis, analyzing the projection of the rod along the image Y-axis, analyzing the projection of the rod along the axis of the rod. Wherein these steps may be sub steps in step S3, or the steps may be included as further method steps between step S3 and step S4. Furthermore, the step S3 of analyzing the projection of the rod along the image X-axis may comprise a step of measuring the projection of the rod in the X-ray image, and/or a step of determining the rods radius and height from the known geometry of the marker. Also the steps may be sub steps of step S3 or may be added as further steps for the method between the steps S3 and step S4. The sub steps of S3 may be further divided into a step of determining a lower rod radius at a base of the rod, determining a higher rod radius at the tip of the rod.

Additional steps, which may be added between the step S3 and the step S4 may be a step of determining a distance from the rod to a line normal to the patient's bed passing through the X-ray source, a step of determining a distance between the patient's bed and a detector.

Further, the step of detecting the marker may comprises a step of a detection of letters arranged at the marker, which may comprise detection of at least two letters arranged at the marker. The step of determining the marker using deep learning method for object detection may comprise in particular a Faster RCNN or YOLO.

Furthermore, the method may comprise the additional step of calculating a relative position of an image structure based on the determined position of the X-ray source relative to the patient.

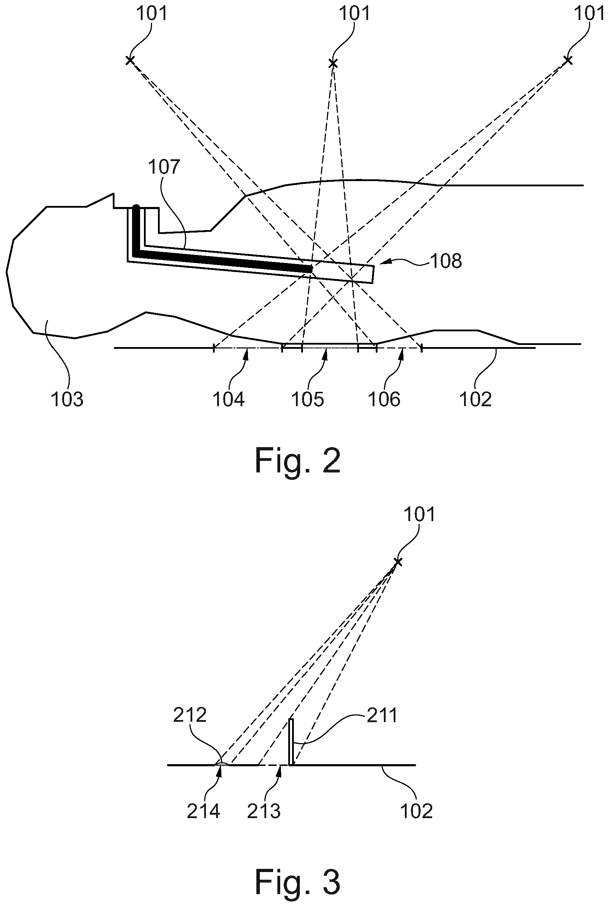

Fig. 2 illustrates different projections of an object 107 in dependence of the position of the X-ray source 101. Fig. 2 shows how a relative position of an endo tracheal tube 107 in the trachea appears different depending on how the X-ray source 101 is positioned relative to the patient 103. The patient 103 is laying above the detector 102 and the X-ray source 101 has to be placed over the patient, wherein the position of the X-ray source101 may vary depending on outer circumferences. If the technician needs to place the X-ray source over the head of the patient, as illustrated on the left side of Fig. 2 this results in a silhouette 106, which is noticeably shorter than if the technician had to place the X-ray source over the patient's belly which is illustrated on the right side of the Fig. 2. The resulted silhouette, when the X-ray source is placed over the belly is illustrated with 104. As can be seen in Fig. 2 depending on the position of the X-ray source 101 the resulting projection of the endo tracheal tube may vary. The respective resulting projection is illustrated with projection 104, projection 105, and projection 106. Those distortions caused by the X-ray source placement, complicate physical measurements. In the case of the endotracheal tube, the physical distance from the trachea bifurcation point (carina) 108 to the endotracheal tube tip 107 is an indicator of the correct position of the tube inside the patient 103. Hence, there exist a need for mobile X-ray systems to eliminate or at least reduce those distortions, which are caused by the different projections 104-106 due to the position of the X-ray source 101 over the patient 103. When using the method as described with the exemplary embodiments herein, this problem can be solved.

Fig. 3 illustrates different projections 213, 214 of different objects 211, 212 using one X-ray source 101. In particular, in Fig. 3 it is illustrated that a low-profile object, as object 212, has a little projection distortion 214, even located further away from the X-ray source 101. Moreover, that a larger object, as object 211, has a large a projection distortion 213, even when located nearer to the X-ray source 121 then the low-profile object 212. Therefore, it is important to know the markers geometry used in the X-ray image for the evaluation of the distortion. Accordingly, a standardized marker having standardized dimensions may be used for easing the determination of the marker geometries. Such a marker is illustrated in the following Fig. 4. Nevertheless, it may also be possible that the method only uses the known geometry of the marker in such a manner that the dimensions of the marker have to be provided to the method, to the system before performing the respective method. This means that it is not essential that standardized markers with standardized dimensions be used, it is only necessary that the dimensions are known and provided for the method.

Fig. 4 illustrates a marker 300 according to an exemplary embodiment of the invention. The marker 300 consist of a square plate 331 made of plastic, in order to minimize the visibility on the resulting X-ray, and of a rod 332 made of metal. The rod 332 is arranged at one side of the plate 331. The dimensions of the plate 331 could be set to 80x80x3 mm. The metallic cylindrical rod 332 is arranged at the center of the plate 331 of the marker 300, wherein the rod 332 comprises a known height and diameter, for instance 80x2,5 mm. The rod 332 extends perpendicular from the plate 331 in a direction away from the plate 331. A space, not illustrated, can be made in the plate 331 in which the rod 332 can be clipped by an elastic element and thus ease transportation to the patients bed. In Fig. 3 the marker 300 comprises four letters 333, of the letter "L". Other letters 333 of the alphabet can be arranged on the marker 300 in a similar manner. The letters 333 are arranged on a side of the plate 331 opposite to the side of the plate 331 at which the rod 332 is arranged at the plate. The dimensions of the marker 300 which should be known for the method are at least one of the height 334 of the marker, the length 335 of the marker, the thickness 339 of the marker plate 331, the length 336, 337 of the letter (for instance of each letter, wherein the different letters should comprise the same dimensions). Further, it should be known, at least one of the rod base 338, the length of the rod 340, the diameter of the rod 341 and the diameter of the base of the rod 342. Each of the four letters 333 are arranged in one corner of the square shaped plate 331 with the same distance to the edges of the plate 331.

It should be noted that the above given dimensions are only exemplary, other dimensions may also be possible.

Fig. 5 illustrates an analysis of a projection of the rod 451 according to an embodiment of the invention. In particular, Fig. 5 shows the appearance of the marker on an X-ray image. The projection of the rod 551 can be decomposed on two axes of the X-ray image. Into the y-axis the proj x and the y-axis the proj y. For an easier understanding all following elucidations will be based on the x-axis projection, a similar deduction may be realized for the y-axis. The projection of the rod in the X-ray image is illustrated by 551, this projection can be divided into the projection on the two axes (x, y). Further, Fig. 5 illustrates a projection of the rod along its direction 552. The method may use the projection of the rod 551 in the x-axis and/or y-axes for determining the position of the rod and accordingly the position of the X-ray source relative to the patient.

Fig. 6 illustrates an analysis of the projection of the rod along an image axis according to an embodiment of the invention. In particular, Fig. 6 illustrates the X-axis projection, wherein p(proj x) is the measured length of the projection of the rod 551, r and h as the respectively radius of the rod and height of the rod, d the distance from the rod to a line normal to the patients bed passing through the X-ray source 101, and a the position of the X-ray source 101 above the patients bed and b the distance between the patients bed and the detector 102. The projection of the rod 551 may be measured by pixel analysis, hence measured pixel size and number of pixels. Using Thales theorem leads to the following equation:

$$\frac{\cdots}{\cdots\cdots} = \frac{\cdots}{\cdot} \qquad (2)$$

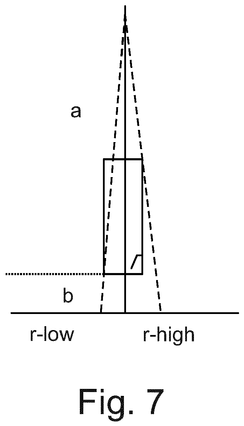

Fig. 7 illustrates an analysis of the projection of the rod 551 along the rod axis according to an embodiment of the invention. In particular, Fig. 7 analyses the rod axis projection with r-low as the lower rod radius at its base and r-high as the higher rod radius at its tip. Because of the projection, the radius at the tip is not equal to the radius at the base of the rod. When now again Thales theorem is used, its leads to the following equation:

$$\frac{\cdot}{\cdot} = \frac{\cdots\cdot}{\cdots} \qquad (3)$$

And in a similar manner for the radius r high:

$$\frac{\cdot}{\cdot} = \frac{\cdots\cdot\cdot}{\cdots} \qquad (4)$$

In the equations (2), (3) and (4) the unknown variables are a, b and d. the physical dimensions h and r are given by the rods dimensions, hence the rod construction. p, r-low and r-high are the physical length of the rod projection which are obtained from the X-ray image using pixel measurement on the X-ray image, which are converted to physical dimensions using a resolution as stored in a DICOM tag (0018,1050). Accordingly, the equations (2), (3) and (4) form an equation system, which can be solved for a, b, and d.

When several markers are used, this allows obtaining several values for the position of the X-ray source and the distance between the patient's bed and the detector such that uncertainties introduced by the pixel measurement of the rods projection can be averaged out.

The method as described with the exemplary embodiments herein, may be able to use the equations for determine the positon of the X-ray source relative to the patient. Accordingly, the method may perform the step of calculating the above mentioned equations.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. It should be noted that the term "comprising" does not exclude other elements or steps and "a" or "an" does not exclude a plurality. Also, elements described in association with different embodiments may be combined.

LIST OF REFERENCE SIGNS:

| | |
|---|---|
| 101 | X-ray source |
| 102 | detector |
| 103 | patient |
| 104 | projection |
| 105 | projection |
| 106 | projection |
| 107 | tube |
| 108 | carina |
| 211 | large object |
| 212 | small object |
| 213 | projection large object |
| 214 | projection small object |
| 300 | marker |
| 331 | plate |
| 332 | rod |
| 333 | letter |
| 334 | height |
| 335 | length |
| 336 | length |
| 337 | length |
| 338 | rod base |
| 339 | thickness of marker |
| 340 | rod length |
| 341 | diameter rod |
| 342 | diameter rod base |
| 551 | projection of the rod |
| 552 | projection along the rods direction |

**Claims**

1. Computer implemented method
   for evaluating a mobile X-ray acquisition of a patient, the method comprising the steps of

   receiving an X-ray image of the patient,
   detecting a marker in the X-ray image using a deep learning method,
   wherein the marker comprises a plate of known geometry and a rod,
   determining a position of the rod of the marker,
   analyzing a projection of the rod in the X-ray image,
   determining a position of the X-ray source above a bed of the patient based on the analyzed projection of the rod.

2. Method according to claim 1,

   wherein the step of analyzing the projection of the rod comprises at least one of the steps of
   analyzing the projection of the rod along the image X-axis,
   analyzing the projection of the rod along the image Y-axis,
   analyzing the projection of the rod along the axis of the rod.

3. Method according to claim 2,

   wherein the step of analyzing the projection of the rod along the image X-axis comprises measuring the projection of the rod in the X-ray image,
   determining the rods radius and height from the known geometry of the marker.

4. Method according to claim 2,

   wherein the step of analyzing the projection of the rod along the axis of the rod comprises determining a lower rod radius at a base of the rod,
   determining a higher rod radius at a tip of the rod.

5. Method according to any one of the preceding claims, further comprises the steps of

determining a distance from the rod to a line normal to the patients bed passing through the X-ray source,

determining a distance between the bed of the patient and a detector.

6. Method according to any one of the preceding claims,
wherein the projection of the rod, the lower radius of the rod, and the higher radius of the rod are measured by pixel measurement on the X-ray image and is converted into physical dimensions using a resolution stored in a DICOM tag.

7. Method according to any one of the preceding claims,
wherein the step of detecting the marker comprises a detection of letters arranged at the marker.

8. Method according to any one of the preceding claims,

wherein the marker comprises four letters arranged at the plate of the marker,
wherein the step of detecting the marker comprises a detection of at least two letters of the marker for determining the position of the rod.

9. Method according to any one of the preceding claims,
wherein the deep learning method uses a deep learning network trained with a dataset of letters on X-rays, wherein the dataset of letters comprises letters in different size and rotation.

10. Method according to any one of the preceding claims, wherein the method further comprises the step of
calculating a relative position of an image structure based on the determined position of the X-ray source relative to the patient.

11. Method according to any one of the preceding claims,

wherein the plate of the marker comprises a square shape,
wherein the rod is arranged at the center of the plate,
wherein at least two letters are arranged on the plate of the marker,
wherein the letters are equal and each letter is arranged in at one edge of the square shaped plate.

12. The method according to any of the preceding claims,
wherein the step of determining the marker using deep learning method for object detection comprises in particular a Faster RCNN or YOLO.

13. System for evaluating a mobile X-ray acquisition, the system comprising,

an X-ray source configured for being positioned above a patient,
a detector configured for detecting X-ray radiation emitted from the X-ray source through the patient,
a marker comprising a plate of known geometry and a rod configured to be positioned between the X-ray source and the detector,
a software module causing a computer system to perform the method of any of claims 1 - 12.

14. Software module causing a computer system to perform a method for evaluating a mobile X-ray acquisition wherein the software module causes the computer to

detect a marker in an X-ray image using a deep learning method,
wherein the marker comprises a plate of known geometry and a rod,
determine a position of the rod of the marker;
analyze a projection of the rod in the X-ray image and
determine a position of the X-ray source above a bed of the patient based on the analyzed projection of the rod.

15. Use of a marker for evaluating a mobile X-ray acquisition, for detecting the marker in an X-ray image using a deep learning method, wherein the marker comprises a plate of known geometry and a rod, the use comprising positioning the marker between the X-ray source and the detector and the steps of determining a position of the rod of the marker; analyzing a projection of the rod in the X-ray image for determining a position of the X-ray source above the patients bed based on the analyzed projection of the rod.

**Patentansprüche**

1. Computerimplementiertes Verfahren zum Auswerten einer mobilen Röntgenaufnahme eines Patienten, wobei das Verfahren Schritte umfasst zum

Empfangen eines Röntgenbildes des Patienten;
Erkennen einer Markierung in dem Röntgenbild unter Verwendung eines Deep-Learning-Verfahrens,
wobei die Markierung eine Platte mit bekannter Geometrie und einen Stab umfasst,

Bestimmen einer Position des Stabes der Markierung,

Analysieren einer Projektion des Stabes in dem Röntgenbild,

Bestimmen einer Position der Röntgenquelle über einem Bett des Patienten basierend auf der analysierten Projektion des Stabes.

2. Verfahren nach Anspruch 1,

wobei der Schritt des Analysierens der Projektion des Stabes zumindest einen der Schritte umfasst zum

Analysieren der Projektion des Stabes entlang der X-Achse des Bildes,

Analysieren der Projektion des Stabes entlang der Y-Achse des Bildes,

Analysieren der Projektion des Stabes entlang der Achse des Stabes.

3. Verfahren nach Anspruch 2,

wobei der Schritt des Analysierens der Projektion des Stabes entlang der X-Achse des Bildes Messen der Projektion des Stabes in dem Röntgenbild umfasst,

Bestimmen des Radius und der Höhe der Stäbe aus der bekannten Geometrie der Markierung.

4. Verfahren nach Anspruch 2,

wobei der Schritt des Analysierens der Projektion des Stabes entlang der Achse des Stabes umfasst

Bestimmen eines unteren Stabradius an einer Basis des Stabes,

Bestimmen eines oberen Stabradius an einer Spitze des Stabes.

5. Verfahren nach einem der vorstehenden Ansprüche, das weiter die Schritte umfasst zum

Bestimmen eines Abstands von dem Stab zu einer Linie normal zum Patientenbett, die durch die Röntgenquelle verläuft,

Bestimmen eines Abstands zwischen dem Bett des Patienten und einem Detektor.

6. Verfahren nach einem der vorstehenden Ansprüche, wobei die Projektion des Stabes, der untere Radius des Stabes und der obere Radius des Stabes durch Pixelmessung auf dem Röntgenbild gemessen, und unter Verwendung einer in einem DICOM-Tag gespeicherten Auflösung in physikalische Abmessungen umgewandelt werden.

7. Verfahren nach einem der vorstehenden Ansprüche, wobei der Schritt des Erkennens der Markierung ein Erkennen von an der Markierung angeordneten Buchstaben umfasst.

8. Verfahren nach einem der vorstehenden Ansprüche,

wobei die Markierung vier auf der Platte der Markierung angeordnete Buchstaben umfasst, wobei der Schritt des Erkennens der Markierung ein Erkennen von zumindest zwei Buchstaben der Markierung zum Bestimmen der Position des Stabes umfasst.

9. Verfahren nach einem der vorstehenden Ansprüche, wobei das Deep-Learning-Verfahren ein Deep-Learning-Netzwerk verwendet, das mit einem Buchstabendatensatz auf Röntgenbildern trainiert wird, wobei der Buchstabendatensatz Buchstaben in unterschiedlicher Größe und Drehung umfasst.

10. Verfahren nach einem der vorstehenden Ansprüche, wobei das Verfahren weiter den Schritt umfasst zum Berechnen einer relativen Position einer Bildstruktur basierend auf der bestimmten Position der Röntgenquelle in Bezug auf den Patienten.

11. Verfahren nach einem der vorstehenden Ansprüche,

wobei die Platte der Markierung eine quadratische Form umfasst,

wobei der Stab im Zentrum der Platte angeordnet ist,

wobei zumindest zwei Buchstaben auf der Platte der Markierung angeordnet sind,

wobei die Buchstaben gleich sind und jeder Buchstabe an einer Kante der quadratisch geformten Platte angeordnet ist.

12. Verfahren nach einem der vorstehenden Ansprüche wobei der Schritt des Bestimmens der Markierung unter Verwendung eines Deep-Learning-Verfahrens zur Objekterkennung insbesondere ein Faster RCNN oder YOLO umfasst.

13. System zum Auswerten einer mobilen Röntgenaufnahme, wobei das System umfasst,

eine Röntgenquelle, die zum Positionieren über einem Patienten konfiguriert ist,

einen Detektor, der zum Erkennen der von der Röntgenquelle durch den Patienten emittierten Röntgenstrahlung konfiguriert ist,

eine Markierung, die eine Platte mit bekannter Geometrie und einen Stab umfasst, der konfiguriert ist, um zwischen der Röntgenquelle und dem Detektor positioniert zu werden,

ein Softwaremodul, das ein Computersystem veranlasst, das Verfahren nach einem der Ansprüche 1 - 12 durchzuführen.

**14.** Softwaremodul, das ein Computersystem veranlasst, ein Verfahren zum Auswerten einer mobilen Röntgenaufnahme durchzuführen, wobei das Softwaremodul den Computer veranlasst,

eine Markierung in einem Röntgenbild unter Verwendung eines Deep-Learning-Verfahrens zu erkennen,
wobei die Markierung eine Platte mit bekannter Geometrie und einen Stab umfasst,
eine Position des Stabes der Markierung zu bestimmen;
eine Projektion des Stabes in dem Röntgenbild zu analysieren und
eine Position der Röntgenquelle über einem Bett des Patienten basierend auf der analysierten Projektion des Stabes zu bestimmen.

**15.** Verwendung einer Markierung zum Auswerten einer mobilen Röntgenaufnahme zum Erkennen der Markierung in einem Röntgenbild unter Verwendung eines Deep-Learning-Verfahrens, wobei die Markierung eine Platte mit bekannter Geometrie und einen Stab umfasst, wobei die Verwendung Positionieren der Markierung zwischen der Röntgenquelle und dem Detektor und die Schritte des Bestimmens einer Position des Stabes der Markierung; Analysieren einer Projektion des Stabes in dem Röntgenbild zum Bestimmen einer Position der Röntgenquelle über dem Patientenbett basierend auf der analysierten Projektion des Stabes umfasst.

**Revendications**

**1.** Procédé mis en œuvre par ordinateur pour évaluer une acquisition radiologique mobile d'un patient, le procédé comprenant les étape de

réception d'une image radiologique du patient,
détection d'un marqueur dans l'image radiologique à l'aide d'un procédé d'apprentissage profond,
dans lequel le marqueur comprend une plaque de géométrie connue et une tige, détermination d'une position de la tige du marqueur,
l'analyse d'une projection de la tige dans l'image radiologique,
détermination d'une position de la source de rayons X au-dessus d'un lit du patient sur la base de la projection analysée de la tige.

**2.** Procédé selon la revendication 1,

dans lequel l'étape d'analyse de la projection de la tige comprend au moins l'une des étapes d' analyse de la projection de la tige le long de l'axe X de l'image,
analyse de la projection de la tige le long de l'axe Y de l'image,
analyse de la projection de la tige le long de l'axe de la tige.

**3.** Procédé selon la revendication 2,

dans lequel l'étape d'analyse de la projection de la tige le long de l'axe X de l'image comprend la mesure de la projection de la tige dans l'image radiologique,
la détermination du rayon et de la hauteur de la tige à partir de la géométrie connue du marqueur.

**4.** Procédé selon la revendication 2,

dans lequel l'étape d'analyse de la projection de la tige le long de l'axe de la tige comprend
la détermination d'un rayon de tige plus petit au niveau d'une base de la tige,
la détermination d'un rayon de tige plus élevé au niveau d'une pointe de la tige.

**5.** Procédé selon l'une quelconque des revendications précédentes, comprenant en outre les étapes de

détermination d'une distance entre la tige et une ligne normale au lit du patient passant par la source de rayons X,
détermination d'une distance entre le lit du patient et un détecteur.

**6.** Procédé selon l'une quelconque des revendications précédentes,
dans lequel la projection de la tige, le rayon plus petit de la tige et le rayon plus élevé de la tige sont mesurés par mesure de pixels sur l'image radiologique et sont convertis en dimensions physiques à l'aide d'une résolution stockée dans une balise DICOM.

**7.** Procédé selon l'une quelconque des revendications précédentes,
dans lequel l'étape de détection du marqueur comprend une détection de lettres agencées au niveau du marqueur.

**8.** Procédé selon l'une quelconque des revendications précédentes,

dans lequel le marqueur comprend quatre lettres agencées sur la plaque du marqueur,
dans lequel l'étape de détection du marqueur comprend une détection d'au moins deux lettres du marqueur pour déterminer la position de la tige.

9.  Procédé selon l'une quelconque des revendications précédentes,
    dans lequel le procédé d'apprentissage profond utilise un réseau d'apprentissage profond entraîné avec un jeu de données de lettres sur des rayons X, dans lequel le jeu de données de lettres comprend des lettres de différentes tailles et rotations.

10. Procédé selon l'une quelconque des revendications précédentes, dans lequel le procédé comprend en outre l'étape de
    calcul d'une position relative d'une structure d'image sur la base de la position déterminée de la source de rayons X par rapport au patient.

11. Procédé selon l'une quelconque des revendications précédentes,

    dans lequel la plaque du marqueur comprend une forme carrée,
    dans lequel la tige est agencée au centre de la plaque,
    dans lequel au moins deux lettres sont agencées sur la plaque du marqueur,
    dans lequel les lettres sont égales et chaque lettre est agencée sur un bord de la plaque de forme carrée.

12. Procédé selon l'une quelconque des revendications précédentes,
    dans lequel l'étape de détermination du marqueur à l'aide d'un procédé d'apprentissage profond pour une détection d'objet comprend, en particulier, un RCNN ou YOLO plus rapide.

13. Système d'évaluation d'une acquisition radiologique mobile, le système comprenant,

    une source de rayons X configurée pour être positionnée au-dessus d'un patient,
    un détecteur configuré pour détecter un rayonnement de rayons X émis par la source de rayons X à travers le patient,
    un marqueur comprenant une plaque de géométrie connue et une tige configurée pour être positionnée entre la source de rayons X et le détecteur,
    un module logiciel amenant un système informatique à réaliser le procédé selon l'une quelconque des revendications 1 - 12.

14. Module logiciel amenant un système informatique à réaliser un procédé d'évaluation d'une acquisition radiologique mobile, dans lequel le module logiciel amène l'ordinateur à

    détecter un marqueur dans une image radiologique à l'aide d'un procédé d'apprentissage profond,
    dans lequel le marqueur comprend une plaque de géométrie connue et une tige, déterminer une position de la tige du marqueur ;
    analyser une projection de la tige dans l'image radiologique et
    déterminer une position de la source de rayons X au-dessus du lit du patient sur la base de la projection analysée de la tige.

15. Utilisation d'un marqueur pour évaluer une acquisition radiologique mobile, pour détecter le marqueur dans une image radiologique à l'aide d'un procédé d'apprentissage profond, dans laquelle le marqueur comprend une plaque de géométrie connue et une tige, l'utilisation comprenant le positionnement du marqueur entre la source de rayons X et le détecteur et les étapes de détermination d'une position de la tige du marqueur ; d'analyse d'une projection de la tige dans l'image radiologique pour déterminer une position de la source de rayons X au-dessus du lit du patient sur la base de la projection analysée de la tige.

X-ray image

S1 — detecting a marker

S2 — determining position of a marker element

S3 — analyzing projection of the marker element

S4 — determining position of the X-ray source

End/Restart

Fig. 1

101 101 101

107

108

103 104 105 106 102

Fig. 2

101

212 211

214 213 102

Fig. 3

Fig. 4

Fig. 5

Fig. 6

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021150474 A1